# EUROPEAN PATENT SPECIFICATION

(11) **EP 1 960 006 B1**
(45) Date of publication and mention of the grant of the patent: **04.11.2009**
(21) Application number: 06818641.0
(22) Date of filing: 17.11.2006
(51) Int. Cl.: A61L 15/46

(54) **ABSORBENT ARTICLES COMPRISING ACIDIC SUPERABSORBER AND AN ORGANIC ZINC SALT**
SAUGFÄHIGE ARTIKEL MIT EINEM SAUREN SUPERABSORBER UND EINEM ORGANISCHEN ZINKSALZ
ARTICLES ABSORBANTS COMPRENANT UN SUPERABSORBANT ACIDE ET UN SEL DE ZINC ORGANIQUE

(30) Priority: 18.11.2005 WO PCT/EP2005/012395
(43) Date of publication of application: 27.08.2008
(73) Proprietor: SCA Hygiene Products AB, 405 03 Göteborg (SE)
(72) Inventor: WÄSTLUND-KARLSSON, Jan, SE-431 59 Mölndal (SE); YHLÉN, Birgitta, SE-435 38 Mölnlycke (SE); KROOK, Patrik, SE- 413 07 Göteborg (SE)
(74) Representative: Wiedemann, Peter
(86) International application number: PCT/EP2006/011073
(87) International publication number: WO 2007/057211

(56) References cited:
- EP-A- 1 532 990
- WO-A-00/35502
- WO-A-97/45013
- DE-A1- 2 900 263
- GB-A- 2 326 348

## Description

The present invention relates to an absorbent article such as a diaper, panty diaper, sanitary napkin or incontinence device comprising an effective odour control system. The present invention relates in particular to such absorbent articles wherein an acidic superabsorbent material and an organic zinc salt such a zinc ricinoleate interact synergetically to reduce malodours such as ammonia.

### TECHNICAL BACKGROUND

One important area of development in the area of absorbent articles of the above-mentioned type is the control of odourous compounds forming typically after the release of body fluids, especially over a longer period of time. These compounds include fatty acids, ammonia, amines, sulphur-containing compounds and ketones and aldehydes. They are present as natural ingredients of body fluids or result from degradation processes of natural ingredients such as urea, which are frequently assisted by microorganisms occurring in the urogenital flora.

Various approaches exist to suppress the formation of unpleasant odours in absorbent articles. WO 97/46188, WO 97/46190, WO 97/46192, WO 97/46193, WO 97/46195 and WO 97/46196 teach for instance the incorporation of odour inhibiting additives or deodorants such as zeolites and silica. The absorption of bodily liquids reduces however the odour inhibiting capacity of zeolites as soon as these become saturated with water, as mentioned for instance in WO 98/17239.

A second approach involves the addition of lactobacilli with the intention of inhibiting malodour-forming bacteria in the product. The incorporation of lactobacilli and their favourable effect are disclosed for instance in SE 9703669-3, SE 9502588-8, WO 92/13577, SE 9801951-6 and SE 9804390-4.

Moreover, it is known from WO 98/57677, WO 00/35503 and WO 00/35505 that partially neutralized superabsorbent materials (acidic superabsorbent materials) counteract the formation of unpleasant odours in absorbent articles. However, acidic superabsorbent materials absorb lower amounts of body fluid compared to regular superabsorbent materials (in the following also referred to as superabsorbent polymer, SAP).

Therefore, an ongoing demand exists in the art for effective odour-control systems in absorbent articles. Specifically, it would be desirable to provide an odour-control system that allows reducing the amount of acidic SAP to be used.

DE 10256569 A1 relates to water-absorbing, crosslinked, acid group-containing polymers in the form of mainly open-cell foam comprising at least one odour control agent selected from compounds with an anhydride group, compounds with an acidic group, cyclodextranes, bactericides and surfactants with an HLB value of less than 12. One example of bactericidal compounds are zinc compounds such as zinc chloride. The open-cell foams according to DE 10256569 A1 to which the odor control agent may be added do not represent acidic superabsorbers. Moreover, no organic zinc salt is mentioned in this reference.

US 2004/0180093 A1 relates to a polymer composition including a hydrophilic amine-containing polymer and a bioactive agent selected from silver, copper and zinc compounds. The examples given for the zinc compound include various inorganic salts, zinc acetate and zinc lactate. This polymer composition is used in medical articles such as wound dressings. Acidic superabsorbers are not mentioned.

US 2004/0024374 A1 is in the name of the present applicant and relates to an absorbent article having a skincare composition applied on at least one portion thereof. One among many examples of skincare substances is zinc ricinoleate. The highly absorbent polymers (SAPs) mentioned in this document are not further specified.

DE 20 2004 015 738 U1 describes an absorbent agent for the reception of humidity and/or odour, **characterized in that** the absorbtion agent consists of a mixture of active carbon and superabsorber. The active carbon may be treated with zinc ricinoleate. Acidic superabsorbers are not mentioned in this document.

DE 199 29 106 A1 pertains to a diaper comprising an absorbent body and an absorbing agent associated therewith **characterized in that** this adsorbing agent is capable of adsorbing ammonia. According to the teaching of this document, this may be achieved by using partially neutralized (acidic) SAPs based on polyacrylic acids. According to one embodiment, active carbon impregnated with zinc salts can also be present. Organic zinc salts are not mentioned in this document.

US 2002/0128621 A1, US 6,503,526 B1 and WO 00/10500 also mention zinc salts in connection with absorbent articles, however as skincare agent in lotions applied on the liquid permeable coversheet thereof.

From other technical areas it is further known that organic zinc salts of unsaturated hydroxylated fatty acids such as zinc ricinoleate are deodorizing active ingredients (see for instance DE 1792074 A1, DE 2548344 A1 and DE 3808114 A1).

It is one technical object of the present invention to overcome deficiencies discussed above in connection with the prior art.

It is one further technical object to provide an absorbent article having an efficient odour control system.

It is one further technical object of the present invention to considerably reduce,or eliminate ammonia formation in absorbent articles.

### BRIEF DESCRIPTION OF THE INVENTION

The present invention relates to an absorbent article, such as a diaper, panty diaper, panty liner, sanitary napkin or incontinence device comprising a liquid-permeable topsheet, a liquid-impermeable backsheet and an absorbent core enclosed between said liquid-permeable topsheet and said liquid-impermeable backsheet, wherein said absorbent core comprises a superabsorbent material, **characterized in that** said superabsorbent material is an acidic superabsorbent material having a pH value of 5.5 or less and the absorbent core additionally comprises an organic zinc salt.

In the present specification, the acidic superabsorbent material (SAP) having a pH value of 5.5 or less is oftentimes simply referred to as acidic superabsorbent material (SAP).

The present inventors have found that acidic superabsorbent material and organic zinc salt, such as zinc ricinoleate interact synergetically in the suppression of unpleasant odours and completed the present invention based on this finding.

### DETAILED DESCRIPTION OF THE INVENTION

Throughout the specification and claims, the use of "comprising" is intended to cover also the more restricting meanings "essentially consisting of" and "consisting".

As "absorbent article" we understand articles capable of absorbing body fluids such as urine, watery feces, female secretion or menstrual fluids. These absorbent articles include, but are not limited to diapers, panty diapers, panty liners, sanitary napkins or incontinence device (as used for instance for adults).

Such absorbent articles have a liquid-pervious topsheet, which during use is facing the wearer's body. They further comprise a liquid-impervious backsheet, for instance a plastic film, a plastic-coated nonwoven or a hydrophobic nonwoven and an absorbent core enclosed between the liquid-pervious topsheet and the liquid-impervious backsheet.

A suitable topsheet may be manufactured from a wide range of materials such as woven and nonwoven materials (e.g. a nonwoven web of fibers), polymeric materials such as apertured plastic films, e.g. apertured formed thermoplastic films and hydroformed thermpoplastic films; porous foams; reticulated foams; reticulated thermoplastic films; and thermoplastic scrims. Suitable woven and nonwoven materials can be comprised of natural fibers (e.g. wood or cotton fibers), synthetic fibers (e.g. polymeric fibers such as polyesters, polypropylene or polyethylene fibers) or from a combination of natural and synthetic fibers. When the topsheet comprises a nonwoven web, the web may be manufactured by a wide number of known techniques. For example, the web may be spun-bonded, carded, wet-laid, melt-blown, hydroentangled, combinations of the above or the like. In accordance with the invention, it is preferred to make use of apertured plastic films (e.g. thermoplastic films) or nonwoven materials based on synthetic fibers, e.g. those made from polyethylene or polypropylene homo- or copolymers and polymer compositions based thereon.

Optionally, at least one further layer exists between the absorbent core and the topsheet and may be made from hydrophobic and hydrophilic web or foam materials. As "web material" we understand coherent flat fiber-based structures of paper tissue, woven or nonwoven type. The nonwoven material may have the same features as described above for topsheets.

Specifically, the at least one further layer may contribute to fluid management, for instance in the form of at least one acquisition/ distribution layer. Such structures are taught for instance by US 5,558,655, EP 0 640 330 A1, EP 0 631 768 A1 or WO 95/01147.

"Foam materials" are also well known in the art and for instance describe in EP 0 878 481 A1 or EP 1 217 978 A1 in the name of the present applicant.

The absorbent core, which may be partially or totally surrounded by a core wrap, comprises an acidic superabsorbent material, optionally in admixture with any other absorbent material that is generally compressible, conformable, nonirritating to the wearer's skin and capable of absorbing and retaining liquids such as urine and other body exudates.

Examples for other absorbent materials include a wide variety of liquid-absorbent materials commonly used in disposable diapers and other absorbent articles such as comminuted wood pulp, which is generally referred to as air felt or fluff, as well as creped cellulose wadding; melt blown polymers, including co-form; chemically stiffened, modified or crosslinked cellulosic fibers; tissue, including tissue wraps and tissue laminates, absorbent foams, absorbent sponges, non-acidic superabsorbent polymers (such as superabsorbent fibers), absorbent gelling materials, or any other known absorbent materials or combinations of materials.

The term "superabsorbent material" is well known in the art and designates water-swellable, water-insoluble materials capable of absorbing the multiple of their own weight in body fluids. Preferably, the superabsorbent material is capable of absorbing at least about 10 times its weight, preferably at least about 15 times its weight, in particular at least about 20 times its weight in an aqueous solution containing 0.9 wt.-% of sodium chloride (under usual measuring conditions where the superabsorbent surface is freely accessible to the liquid to be absorbed). To determine the absorption capacity of the superabsorbent material, the standard test EDANA WSP 241.2 can be used.

The superabsorbent material may be in any form suitable for use in absorbent articles including particles, fibers, flakes, spheres and the like, the particle form being preferred.

Acidic SAPs are based on homo- or copolymers comprising at least one polymerizable unit having an acidic group (e.g. a carboxylic acid group or a sulfonic acid group) such as methacrylic acid, acrylic acid, maleic acid, vinylsulfonic acid. The corresponding polymers include, but are not limited to poly(meth)acrylic acids, ethylene maleic anhydride copolymers, polymers and copolymers of vinylsulfonic acids, polyacrylates, acrylic acid grafted starch and isobutylene maleic anhydride copolymers. These polymers are preferably crosslinked to render the materials substantially water insoluble. According to one preferred embodiment of the present invention, the superabsorbent material is a crosslinked homo- or copolymer comprising (meth)acrylic acid units, for instance of the type disclosed in EP 0 391 108 A2.

The acidic superabsorber has preferably a pH value of 3.0 to 5.5, more preferably 3.5 to 5.3 and most preferably 4.1 to 5.2. Thereby, the pH is measured using the standard test EDANA WSP 200.2.

Unlike standard SAPs having a pH which lies e.g. in a range of 5.8 or more, the acidic SAPs to be used in the present invention have a pH of 5.5 or less, with the preferred ranges as defined in the preceding paragraph.

There are two ways of manufacturing acidic SAP. One way is to add an acid, e.g. citric acid, to a standard SAP, thereby reducing the pH. The other method is to maintain a low degree of neutralisation. A standard SAP has a high percentage (typically at least 70%) of the acidic groups neutralised under formation of alkali metal salts. In contrast thereto, acidic SAPs manufactured according to this method have a lower degree of neutralisation, typically 15 to 60%. The degree of neutralisation and pH strongly correlate which implies that the acidity of the SAP can be controlled by the degree of neutralisation.

In view of the above, it is also preferred that the absorbent core comprising the acidic superabsorbent has a pH value of 3.0 to 5.7, more preferably 3.5 to 5.5, in particular 4.1 to 5.4 after wetting with synthetic urine.

The pH of the absorbent core can be measured very precisely with the following method involving the preparation of a test absorbent core and pH measurement using the same.

### Method 1: Preparation of absorbent cores for test

Absorbent cores were punched out of an absorbent core produced in a pilot plant. A standard method of mat forming a core was used in the production of the core in the pilot plant. The absorbent core consisted of a homogenous mixture of fluffed pulp and superabsorbent material. The absorbent core was compressed to a bulk of about 8-10 cm³/g. The size of the punched cores was 5 cm in diameter, the weight of the same about 1.2 g.

### Method 2: Measurement of pH in an absorbent core

An absorbent core having a diameter of approximately 50 mm was prepared according to Method 1. A predetermined amount of Test liquid 1 was added, 16 ml to all samples, whereafter the absorbent core was left to swell for 30 minutes. Thereafter, pH was measured on the liquid squeezed out of the samples using a surface electrode, Flat-bottomed, type Single Pore Flat, Hamilton.

### Test liquid 1 (referred to in Method 2):

synthetic urine containing the following substances: KC1, NaCl, MgSO₄, KH₂PO₄, Na₂HPO₄, NH₂CONH₂. The pH in this composition is 6.0 ± 0.5.

The test liquid to be used is 16 ml synthetic urine (as defined above) for a single core absorbent body. The absorbent core preferably comprises at least one layer comprising a mixture of fibers and acidic superabsorbent material, organic zinc salt and optionally non-acidic superabsorbent material.

As non-acidic superabsorbent material (also designated in the present specification as standard superabsorbent material) we understand superabsorbent materials of the above described type showing a pH of e.g 5.8 or more. Non-acidic SAPs comprising polymerizable units with acidic groups preferably have a neutralization degree of at least 70%.

The fibers present in the absorbent core are preferably also capable of absorbing body liquid as is the case for hydrophilic fibers. Most preferably the fibers are cellulosic fibers such as woodpulp fluff, cotton, cotton linters, rayon, cellulose acetate and the like, the use of cellulosic fluff pulp being preferred. The cellulosic fluff pulp can be of mechanical or chemical type, the chemical pulp being preferred.

In the absorbent core and, if applicable, each layer thereof, the total amount of superabsorbent material (of acidic and optionally non-acidic type) is preferably 10 to 70 wt.-%, more preferably 20 to 65 wt.-%, in particular 30 to 60 wt.-%, for instance 30 to 50 wt.-%, based on the weight of the entire mixture of fibers and superabsorbent materials (without organic zinc salt).

The weight ratio of acidic SAP/non-acidic SAP is not particularly restricted (e.g. 5/95 to 95/5, 10/90 to 90/10, 20/80 to 80/20), even though it would appear that higher amounts of acidic SAP seem to enhance the effect of the present invention. Accordingly, weight ratios of acidic SAP/non-acidic SAP of 100/0 to 50/50 (e.g. 95/5 to 60/40, 90/10 to 70/30) can be preferably selected depending on the properties to be achieved.

Very low amounts of organic zinc salts cooperate already with acidic SAP in a very efficient odour control. A preferred lower weight limit of organic zinc salt (calculated as zinc) seems to be at least 10⁻⁵g per g dry acidic SAP. Herein the term "dry" used in relation to acidic SAP is to be understood such that no water has been added to the acidic SAP and that the only water present in the acidic SAP is the unavoidable residual water from manufacturing. More preferably, the organic zinc salt is present in amounts of at least 10⁻⁴ g, even more preferably at least 5 x 10⁻⁴g, even more preferably at least 10⁻³g. According to the most preferred embodiment, the amount of the zinc salt is at least 10⁻²g zinc per g dry acidic SAP. There is no specific upper limit, even though for economic reasons, a point may be reached where it may no longer be useful to further increase the zinc content, for instance to values of 0,1 or 1 g zinc per g acidic SAP, if this is not accompanied by an enhanced odour suppression.

For instance, when 40% acidic SAP is present in the absorbent core, the concentration of the organic zinc salt may be in the range of 0.03 to 30 weight percent.

There are also no specific restrictions regarding the organic zinc salt to be used. In accordance with the present invention, zinc salts of organic carboxylic acids having 2 to 30 carbon atoms, in particular 12 to 24 carbon atoms are preferably used. The carboxylic acid group may be attached to aliphatic, aliphatic-aromatic, aromatic-aliphatic, alicyclic, or aromatic residues, wherein the aliphatic chain or the alicyclic ring(s) may be unsaturated and are optionally substituted, for instance by hydroxy or C1 to C4 alkyl. These salts include zinc acetate, zinc lactate, zinc ricinoleate and zinc abietate. More preferably, the zinc salt is the zinc salt of an unsaturated hydroxylated fatty acid having 8 to 18 carbon atoms. Although there is no specific restriction regarding the number of unsaturated double bonds or hydroxy groups, those fatty acids having one or two unsaturated double bonds and one or two hydroxyl groups seem to be preferred. The most preferred embodiment is zinc ricinoleate.

According to one embodiment of the present invention the above-described organic zinc salt, such as zinc ricinoleate is activated by an amino acid as in the commercially available TEGO^{®} Sorb A30 (Degussa).

The organic zinc salt to be used in the present invention may also be capable of removing malodorous substances chemically based on amines, *e*.*g*., nicotine in cigarette smoke, thiocompounds, *e*.*g*., allicin in garlic and onions, and acids, *e*.*g*., isovaleric acid in human sweat, and butyric acid. For instance, zinc ricinoleate which is, *e*.*g*., marketed by Degussa under the tradename TEGO^{®} Sorb has the described additional odor removing effect apart from removing ammonia.

The present invention is also not subject to any limitations regarding the technique of incorporating the organic zinc salt into the absorbent core. Dipping and spraying are preferred.

For instance, it is conceivable to treat the fibers present in the absorbent core, preferably cellulosic fluff pulp with a solution of the organic zinc salt prior to or during admixture with the acidic SAP.

According to a preferred embodiment, a solution of the organic zinc salt is sprayed onto the fibers, most preferably onto the cellulosic fluff pulp sheets as obtained from the manufacturer. The organic zinc salt solution can be sprayed onto the fluff pulp sheet directly by the manufacturer of these sheets prior to the delivery of the sheets to the manufacturer of the absorbent articles. This is an especially preferred embodiment since it avoids the extra step of spraying the organic zinc salt solution when manufacturing the absorbent article. Alternatively, the fibers are dipped into the solution. The acidic SAP is then added during or after core formation.

Alternatively, a core is formed by means of conventional techniques from the fibers, preferably cellulosic fluff, said core being sprayed during core formation with the zinc salt solution. The acidic SAP is incorporated either during or after core formation, though spraying the already formed absorbent core is less preferred.

From the presently available information, it would however appear that the most effective technique of achieving zinc incorporation involves treating an already formed absorbent core comprising a mixture of acidic SAP, fibers, preferably cellulosic fluff pulp and optionally non-acidic SAP with a solution of the organic zinc salt, in particular the zinc ricinoleate solution.

According to one preferred application technique, the solution of the organic zinc salt, in particular zinc ricinoleate is sprayed on one or both sides of the absorbent core, or one of both sides of the individual layers constituting the same.

The solvent used for this solution can be water, a preferably volatile organic solvent such as ethanol or a mixture of water and a water-miscible organic solvent such as ethanol. Preferably, the organic zinc solvent is present in the solution in a relatively high concentration, preferably 1 to 30 wt.-%. The use of such concentrated solutions ensures that the absorption capacity of the superabsorbent material is not impaired more than necessary. Commercially available solutions of organic zinc salts such as TEGO^{®} Sorb A30 available from Degussa (content of actives 30 weight%, zinc ricinoleate activated by an amino acid) can also be employed.

The backsheet prevents the exudates absorbed by the absorbent layer and containing with the article from soiling other external articles that may contact the absorbent article, such as bed sheets and undergarments. In preferred embodiments, the backsheet is substantially impervious to liquids (e.g., urine) and comprises a laminate of a nonwoven and a thin plastic film such as a thermoplastic film having a thickness of about 0.012 mm to about 0.051 mm. Suitable backsheet films include those manufactured by Tredegar Industries Inc. of Terre Haute, Ind. and sold under the trade names X15306, X10962, and X10964. Other suitable backsheet materials may include breathable materials that permit vapors to escape from the absorbent article while still preventing exudates from passing through the backsheet. Exemplary breathable materials may include materials such as woven webs, nonwoven webs, composite materials such as film-coated nonwoven webs, and microporous films.

The above elements of an absorbent article can be assembled, optionally together with other typical elements of absorbent articles in a manner known in the art.

The following examples and comparative examples illustrate the present invention.

### EXAMPLE 1

Circular test absorbent cores having a weight of about 1,2 g and a diameter of 5 cm were punched out of an absorbent core produced in a pilot plant. A standard method of mat forming a core was used in the production of the core in the pilot plant. The absorbent core consisted of a homogenous mixture of fluff pulp and superabsorbent material. The fluff pulp used was 0.72 g Weyerhauser pulp (NB 416) and the superabsorbent material was 0.48 g of an acidic superabsorber (pH 5.1) named Z3106 from Degussa. The absorbent core was compressed to a bulk of about 8-10 cm³/g.

To the absorbent core 1.3 ml of a 3 wt.-% solution of zinc ricinoleate (available from Degussa under the tradename TEGO^{®} Sorb A30, diluted 1/10) was added by either dripping the solution onto the surface (on one side) or dipping one side of the core into the solution. 1 week after the treatment, the absorbent body was allowed to absorb 16 ml synthetic urine according to Method 3 as described below and then allowed to stand at room temperature.

4 h, 6h and 8h after the absorption of synthetic urine the amount of ammonia developed was measured.

Five measurements were averaged as mean value. The results are shown in Table 1.

### Method 3: Measurement of ammonia inhibition in absorbent cores

Absorbent cores were prepared in accordance with Method 1. Test liquid 2 was prepared. Bacteria suspension of Proteus mirabilis was cultivated in nutrient broth 30°C overnight. The graft cultures were diluted and the bacterial count was determined. The final culture contained approximately 10⁵ organisms per ml of test liquid. The absorbent core was placed in a plastic jar and the Test liquid 2 was added to the absorbent core, whereafter the container was incubated at 35°C for 4, 6, and 8 hours respectively, whereafter samples were taken from the containers using a hand pump and a so called Dräger-tube. The ammonia content was obtained as a colour change on a scale graded in ppm or volume percent.

### Test liquid 2:

Sterile synthetic urine to which has been added a growth medium for micro-organisms. The synthetic urine contains mono- and divalent cations and anions and urea and has been prepared in accordance with the information in Geigy, Scientific Tables, Vol 2, 8th ed. 1981 p. 53. The growth medium for the micro-organisms is based on information of Hook- and FSA-media for entero-bacteria. The pH in this mixture is 6.6.

### EXAMPLE 2

An absorbent core was formed in the same manner as in Example 1 with the sole exception that the aqueous solution added to the absorbent core, either by dripping the solution onto the surface (on one side) or dipping one side of the core into the solution, contained 6 wt.-% of zinc ricinoleate.

### EXAMPLE 3

An absorbent core was formed in the same manner as in Example 1 with the sole exception that the aqueous solution added to the absorbent core, either by dripping the solution on to the surface (on one side) or dipping one side of the core into the solution, contained 0.5 wt.-% of zinc ricinoleate.

### EXAMPLE 4

An absorbent core was formed in the same manner as in Example 1 with the sole exception that the aqueous solution added to the absorbent core, either by dripping the solution on to the surface (on one side) or dipping one side of the core into the solution, contained 0.3 wt.-% of zinc ricinoleate.

### REFERENCE EXAMPLE

An absorbent body was formed in the same manner as in example 1 with the sole exception that the acidic superabsorber was substituted for a conventional non-acidic superabsorber ( XZS 91030.03 available from Dow Chemicals). Moreover, no treatment with zinc ricinoleate was carried out.

### COMPARATIVE EXAMPLE 1

An absorbent core was formed in the same manner as in Example 1, with the sole exception that the acidic superabsorber was replaced by a non acidic type (Dow XZS 91030.03, available from Dow chemicals).

### COMPARATIVE EXAMPLE 2

An absorbent body was formed in the same manner as in Example 1 with the sole difference that a treatment with a solution of zinc ricinoleate was not carried out.

The results in terms of ammonia formation of the reference example, comparative Examples 1 and 2 and Examples 1 to 4 are shown in the following Table 1.

**TABLE 1**

| | | | ammonia formation (ppm) | | |
|---|---|---|---|---|---|
| | sample description | Zn (10-³g Zn /g a-SAP) | 4 h | 6 h | 8 h |
| Ref. | pulp + SAP¹ | - | < 3 | > 480 | > 1100 |
| Cex 1 | pulp + Zn³+ SAP¹ | 16,1 | < 2 | 19 | 270 |
| CEx 2 | pulp + a-SAP² | - | | 26 | 490 |
| Ex 1 | pulp + Zn³+ a-SAP² | 8,0 | | < 2 | < 2 |
| Ex 2 | pulp + Zn³+ a-SAP² | 16,1 | | 0 | < 2 |
| Ex 3 | pulp + Zn³+ a-SAP² | 1,33 | | 0 | 2 |
| Ex 4 | pulp + Zn³+ a-SAP² | 0,8 | | 1 | 3 |

| | | | | | |
|---|---|---|---|---|---|
| ¹ Non-acidic superabsorber (Dow Chemicals, XZS 91030.03) ² Acidic superabsorber (Z3106, Degussa). ³ Zinc ricinoleate | | | | | |

### EXAMPLE 5

An absorbent core was formed in the same manner as in Example 1 with the sole exception that the commercially available acidic superabsorber M7125 (BASF, pH = 4.9 ± 0,2) was used instead of Z3106 from Degussa.

### COMPARATIVE EXAMPLE 3

An absorbent core was formed in the same manner as in Example 5 with the sole exception that no zinc ricinoleate was added thereto.

The results in terms of ammonia formation of Comparative Example 3 and Example 5 are shown in the following table 2.

**TABLE 2**

| | | | ammonia formation (ppm) | | |
|---|---|---|---|---|---|
| | sample description | Zn (10-³g Zn /g a-SAP) | 6 h | 8 h | 10,5 h |
| Comparative Example 3 | pulp + a-SAP¹ | - | 17 | 180 | 640 |
| Example 5 | pulp + Zn²+ a-SAP¹ | 8,0 | 0 | < 1 | < 4 |

| | | | | | |
|---|---|---|---|---|---|
| ¹ acidic superabsorber (M7125, BASF) ² Zinc ricinoleate | | | | | |

The above experiments show that the combined use of an acidic superabsorber and an organic zinc salt such as zinc ricinoleate suppresses the formation of ammonia to a very surprising extent. Considering the fact that a human can detect the smell of ammonium at a concentration of 30 ppm, the present invention ensures that during use of an absorbent article, no ammonia odour will be perceived by the wearer.

## Claims

1. Absorbent article, such as a diaper, panty diaper, sanitary napkin or incontinence device comprising a liquid-permeable topsheet, a liquid-impermeable backsheet and an absorbent core enclosed between said liquid-permeable topsheet and said liquid-impermeable backsheet, wherein said absorbent core comprises a superabsorbent material, **characterized in that** said superabsorbent material is an acidic superabsorbent material having a pH of 5.5 or less and the absorbent core additionally comprises an organic zinc salt.

2. Absorbent article according to claim 1, wherein the absorbent core comprises a mixture of fibers, in particular cellulosic fluff pulp, acidic superabsorbent material and optionally non-acidic superabsorbent material.

3. Absorbent article according to claim 1 or 2 wherein the total amount of acidic and non-acidic superabsorbent material is 10 to 70 weight%, based on the weight of the core excluding the organic zinc salt.

4. Absorbent article according to claim 2 or 3 wherein the acidic superabsorber is present in an amount of at least 50 weight% based on the total amount of superabsorbent materials.

5. Absorbent article according to any of claims 1 to 4, wherein the amount of organic zinc salt is at least 10⁻⁵g Zn per g dry acidic superabsorbent material.

6. Absorbent article according to any of claims 1 to 5, being obtainable by treating the absorbent core with a solution of the organic zinc salt.

7. Absorbent article according to any of claims 1 to 6, wherein the absorbent core has a pH value of 3.0 to 5.7, preferably 3.5 to 5.5, in particular 4.1 to 5.4 after wetting with synthetic urine.

8. Absorbent article according to any of claims 1 to 6, wherein the acidic superabsorbent material has pH value of 3.0 to 5.5, preferably 3.5 to 5.3 and more preferably 4.1 to 5.2.

9. Absorbent article according to any of claims 1 to 8 wherein the acidic superabsorbent material is a crosslinked homo- or copolymer comprising (meth)acrylic acid units.

10. Absorbent article according to any of the preceding claims, wherein the organic zinc salt is selected from zinc salts of carboxylic acids having 2 to 30 carbon atoms.

11. Absorbent article according to claim 10 wherein the carboxylic acid represents an unsaturated hydroxylated fatty acid having 8 to 18 carbon atoms.

12. Absorbent article according to claim 11 wherein the zinc salt is zinc ricinoleate.

## Patentansprüche

1. Absorbierender Gegenstand, wie eine Windel, Hosenwindel, Damenbinde oder Inkontinenzvorrichtung, die ein flüssigkeitsdurchlässiges Topsheet, ein flüssigkeitsundurchlässiges Backsheet und einen zwischen dem flüssigkeitsdurchlässigen Topsheet und dem flüssigkeitsundurchlässigen Backsheet eingeschlossenen absorbierenden Kern umfasst, wobei der absorbierende Kern ein superabsorbierendes Material umfasst, **dadurch gekennzeichnet, dass** das superabsorbierende Material ein saures superabsorbierendes Material mit einem pH von 5,5 oder weniger ist und der absorbierende Kern zusätzlich ein organisches Zinksalz umfasst.

2. Absorbierender Gegenstand gemäß Anspruch 1, in dem der absorbierende Kern eine Mischung von Fasern, insbesondere Zellulose-Fluff-Zellstoff, saures superabsorbierendes Material und optional nicht-saures superabsorbierendes Material umfasst.

3. Absorbierender Gegenstand gemäß Anspruch 1 oder 2, in dem die Gesamtmenge von saurem und nicht-saurem superabsorbierenden Material 10 bis 70 Gew.%, bezogen auf das Gewicht des Kerns, ausgenommen das organische Zinksalz, ist.

4. Absorbierender Gegenstand gemäß Anspruch 2 oder 3, in dem der saure Superabsorber in einer Menge von mindestens 50 Gew.%, bezogen auf die Gesamtmenge superabsorbierender Materialien, vorliegt.

5. Absorbierender Gegenstand gemäß mindestens einem der Ansprüche 1 bis 4, in dem die Menge organisches Zinksalz mindestens 10⁻⁵g Zn pro g trockenes saures superabsorbierendes Material ist.

6. Absorbierender Gegenstand gemäß mindestens einem der Ansprüche 1 bis 5, der erhältlich ist durch Behandeln des absorbierendes Kerns mit einer Lösung des organischen Zinksalzes.

7. Absorbierender Gegenstand gemäß mindestens einem der Ansprüche 1 bis 6, in dem der absorbierende Kern nach dem Befeuchten mit synthetischem Urin einen pH-Wert von 3,0 bis 5,7, vorzugsweise 3,5 bis 5,5, insbesondere 4,1 bis 5,4 hat.

8. Absorbierender Gegenstand gemäß mindestens einem der Ansprüche 1 bis 6, in dem das saure superabsorbierende Material einen pH-Wert von 3,0 bis 5,5, vorzugsweise 3,5 bis 5,3 und mehr bevorzugt 4,1 bis 5,2 hat.

9. Absorbierender Gegenstand gemäß mindestens einem der Ansprüche 1 bis 8, in dem das saure superabsorbierende Material ein vernetztes Homo- oder Copolymer ist, das (Meth)acrylsäureeinheiten umfasst.

10. Absorbierender Gegenstand gemäß mindestens einem der vorhergehenden Ansprüche, in dem das organische Zinksalz ausgewählt ist aus Zinksalzen von Carbonsäuren mit 2 bis 30 Kohlenstoffatomen.

11. Absorbierender Gegenstand gemäß Anspruch 10, in dem die Carbonsäure eine ungesättigte hydroxylierte Fettsäure mit 8 bis 18 Kohlenstoffatomen bezeichnet.

12. Absorbierender Gegenstand gemäß Anspruch 11, in dem das Zinksalz Zinkricinoleat ist.

## Revendications

1. Article absorbant, tel qu'une couche, une couche-culotte, une serviette hygiénique ou une protection anti-incontinence, qui comprend une feuille de dessus, perméable aux liquides, une feuille de dessous, imperméable aux liquides, et un coeur absorbant enfermé entre ladite feuille de dessus perméable aux liquides et ladite feuille de dessous imperméable aux liquides, et dans lequel article ledit coeur absorbant comprend un matériau superabsorbant, **caractérisé en ce que** ce matériau superabsorbant est un matériau superabsorbant acide présentant un pH inférieur ou égal à 5,5 et **en ce que** le coeur absorbant comprend en outre un sel organique de zinc.

2. Article absorbant conforme à la revendication 1, dans lequel le coeur absorbant comprend un mélange de fibres, en particulier de la pâte cellulosique de type fluff, et un matériau superabsorbant acide, et en option, un matériau superabsorbant non-acide.

3. Article absorbant conforme à la revendication 1 ou 2, dans lequel la quantité totale des matériaux superabsorbants, acide et non-acide, représente de 10 à 70 % du poids du coeur, sel organique de zinc exclu.

4. Article absorbant conforme à la revendication 2 ou 3, dans lequel le matériau superabsorbant acide se trouve en une quantité qui représente au moins 50 % du poids total des matériaux superabsorbants.

5. Article absorbant conforme à l'une des revendications 1 à 4, dans lequel la quantité de sel organique de zinc équivaut à au moins 10⁻⁵ gramme de zinc par gramme de matériau superabsorbant acide sec.

6. Article absorbant conforme à l'une des revendications 1 à 5, qu'on peut obtenir en traitant le coeur absorbant avec une solution du sel organique de zinc.

7. Article absorbant conforme à l'une des revendications 1 à 6, dans lequel le coeur absorbant présente, après mouillage avec de l'urine synthétique, un pH de 3,0 à 5,7, de préférence de 3,5 à 5,5 et en particulier de 4,1 à 5,4.

8. Article absorbant conforme à l'une des revendications 1 à 6, dans lequel le matériau superabsorbant acide présente un pH de 3,0 à 5,5, de préférence de 3,5 à 5,3 et mieux encore de 4,1 à 5,2.

9. Article absorbant conforme à l'une des revendications 1 à 8, dans lequel le matériau superabsorbant acide est un homopolymère ou copolymère réticulé qui comporte des motifs dérivés de l'acide acrylique ou de l'acide méthacrylique.

10. Article absorbant conforme à l'une des revendications précédentes, dans lequel le sel organique de zinc est choisi parmi les sels de zinc des acides carboxyliques comportant de 2 à 30 atomes de carbone.

11. Article absorbant conforme à la revendication 10, dans lequel l'acide carboxylique est un acide gras insaturé hydroxylé comportant de 8 à 18 atomes de carbone.

12. Article absorbant conforme à la revendication 11, dans lequel le sel organique de zinc est du ricinoléate de zinc.
